# EUROPEAN PATENT SPECIFICATION

(11) **EP 0 374 542 B1**
(45) Date of publication and mention of the grant of the patent: **09.11.1994**
(21) Application number: 89122026.1
(22) Date of filing: 29.11.1989
(51) Int. Cl.: A41B 13/04, A61F 5/44

(54) **Improved brief**
Unterhose
Slip

(30) Priority: 20.12.1988 US 287926
(43) Date of publication of application: 27.06.1990
(73) Proprietor: THE PROCTER & GAMBLE COMPANY, Cincinnati, Ohio 45202 (US)
(72) Inventor: Jürgens, Stanley Georges, Cincinnati Ohio 45240 (US)
(74) Representative: Bottema, Johan Jan

(56) References cited:
- FR-A- 2 554 714
- LU-A- 75 162
- US-A- 4 338 939
- US-A- 4 490 148

## Description

This invention is directed to garments worn to assist in the collection of bodily discharges and more particularly to garments which hold a separate and removable absorbent core.

### BACKGROUND OF THE INVENTION

Garments and related articles which protect against incontinence are well known in the art. These garments typically have a body portion which holds or otherwise positions an absorbent core against the body of the wearer. Many garments have structural features which also hold the absorbent core in a desired position within the body portion of the garment until the core is soiled and discarded.

For example, U.S. Patent No. 3,452,753, issued to Sanford on July 1, 1969 discloses an incontinence device having parallel longitudinal side flaps which overlay a separate insertable and removable core and retain it in the desired position. Between the flaps is a rectangularly shaped urine acquisition zone. This concept is extended in U.S. Patent No. 3,771,524, issued to Ralph on November 13, 1973 which discloses a diaper garment having parallel longitudinal side flaps interleaved with the core to prevent it from slipping or moving relative to the diaper garment. Another variant is shown in U.S. Patent No. 4,490,148 issued to Beckestrom on December 25, 1984 which teaches an incontinence protector having longitudinal side flaps that overlay an absorbent core and are elasticized to encourage the side flaps to seal against the thigh creases of the wearer. A feature common to each of these references is that the flaps are mutually parallel.

A napkin structure in which the flaps are not mutually parallel but are inclined to one another, so as to create a tapering space therebetween along the length of the napkin, is disclosed in French Patent No. 2554714 to Boussac Saint Freres BSF. This napkin structure is however integral, such that the absorbent body is not removable from the outer sheets that cover it, is unelasticised and is designed to be held in place by an undergarment rather than being fastened about the waist of a wearer.

Several attempts have been made in the art to provide an irregular or somewhat elliptically shaped opening, or urine acquisition zone, between the side flaps. U.S. Patent No. 2,575,164, issued to Donovan on November 13, which discloses the features appearing in the pre-amble of claim 1, 1951 and U.S. Patent No. 3,593,716, issued to Vogt on July 20, 1971 disclose diapers having generally elliptically shaped holes in the topsheet. However, these references do not teach a garment having a urine acquisition zone of optimum width and position to accommodate bodily discharges while providing a high degree of core retention.

To provide optimum comfort and protection to the wearer, the flaps must balance opposing criteria. The flaps must laterally overlay the core a distance sufficient for frictional engagement to prevent relative movement of the core, but be spaced far enough from the other flap to provide a urine acquisition zone of sufficient width, to prevent wetting of the flaps and subsequent contact of the urine with the skin of the wearer. If the flap does not sufficiently overlay the core, i.e. is too widely spaced from the other flap, movement of the core may result and the core may even become detached from the garment. Either occurrence causes discomfort to the wearer. If the flaps are too closely spaced, the core will be more securely held in position, but a urine target zone of insufficient width results.

Separate fastening means, such as adhesive and mechanical fasteners, may be utilized to hold the core in the desired position within the garment. However, such fastening means are costly and may interfere with removal and insertion of the core, particularly if the wearer has limited manual dexterity.

### BRIEF SUMMARY OF THE INVENTION

It is an object of this invention to provide a garment which holds an absorbent core in place within the garment, without the need for separate or additional fastening means. It is also an object of this invention to provide a garment which optimizes the necessities of having both a large urine acquisition zone and flaps which adequately hold the core in position.

According to the present invention there is provided a garment for holding a separate and removable absorbent core, said garment having a longitudinal centerline, and comprising:
a center panel, generally symmetrically disposed about the longitudinal centerline and having a greater longitudinal than lateral dimension, said center panel being formed with a front waist margin and a rear waist margin;
two oppositely disposed flaps extending longitudinally of the center panel from the front waist margin to the rear waist margin, each said flap having a proximal edge and a distal edge, said proximal edge being affixed to a longitudinal side of said center panel, said distal edge being disposed towards the longitudinal centerline whereby the lateral distance from the centerline to said distal edge is less than the lateral distance from the centerline to said proximal edge, said distal edge being tri-sectional, with a central section disposed intermediate two longitudinally outer sections, each said flap optionally being provided along its distal edge with elastic means along at least the central section;
wherein said garment is of generally quadrilateral shape and is formed with integral means for securing said garment about the waist of a wearer, and in that the distal edge of each said flap is formed so as to be convergent monotonically with the longitudinal center line over at least one of its sections in a direction towards the rear waist margin, the distal edge of any section not so convergent being disposed parallel to the longitudinal outer line.

### BRIEF DESCRIPTION OF THE DRAWINGS

While the Specification concludes with claims particularly pointing out and distinctly claiming the present invention, it is believed that the same will be better understood from the following description taken in conjunction with the accompanying drawings, wherein like parts are given the same reference numeral and related part are designated by one or more prime symbols:
- Figure 1: is a top plan view of an embodiment of the present invention having no elastic induced contraction;
- Figure 2: is a vertical sectional view taken along line 2-2 of Figure 1;
- Figure 3: is a perspective view of the embodiment of Figure 1 when ready to be secured to a wearer (not shown); and
- Figure 4: is a top plan view of second and third embodiments, each having no elastic induced contraction, the embodiment of Figure 4a having flaps which taper outwardly near the front waist margin of the garment and the embodiment of Figure 4b having flaps which taper inwardly near the rear waist margin of the garment.

### DETAILED DESCRIPTION OF THE INVENTION

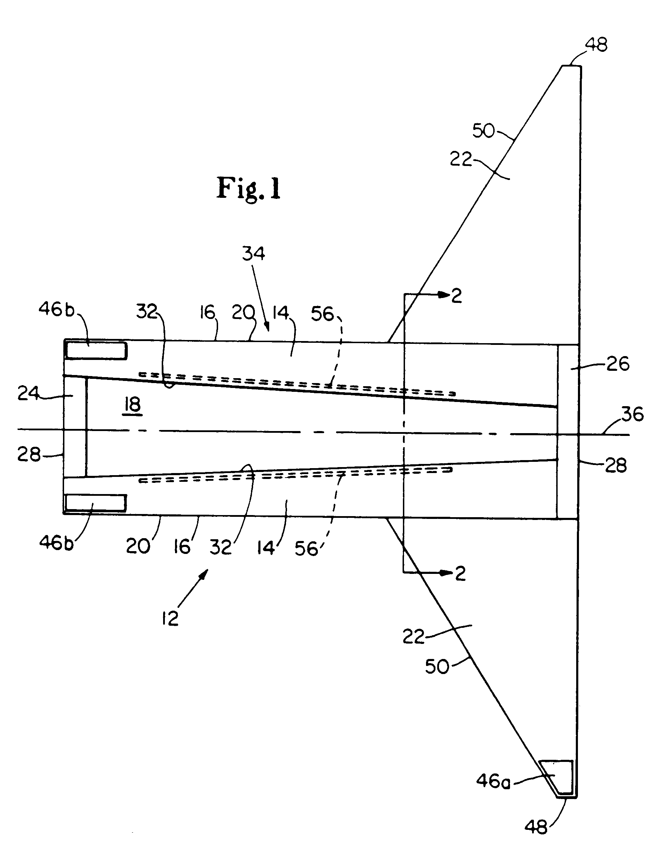

As shown by Figure 1, the invention comprises a durable garment 12 which holds a removable absorbent core 30 (not shown). The garment 12 features two flaps 14, or leg cuffs, which may extend substantially the longitudinal dimension of the garment 12. The central portion of the flaps 14 may be elasticized to form a barrier leg cuff and seal the garment 12 more closely to the wearer. The proximal edges 16 of the flaps 14 are joined to a center panel 18 which forms the bottom of the garment 12. Projecting from each longitudinal side 20 of the center panel 18 near the rear waist margin 26 is a wing 22 which is adjoined with the opposing wing 22 to encircle the waist of the wearer. Elastics for the front and rear waist margins 24 and 26 respectively are provided at the transverse edges 28 of the garment 12. The elastic of the rear waist margin 26 provides adjustment to fit various sizes of wearers.

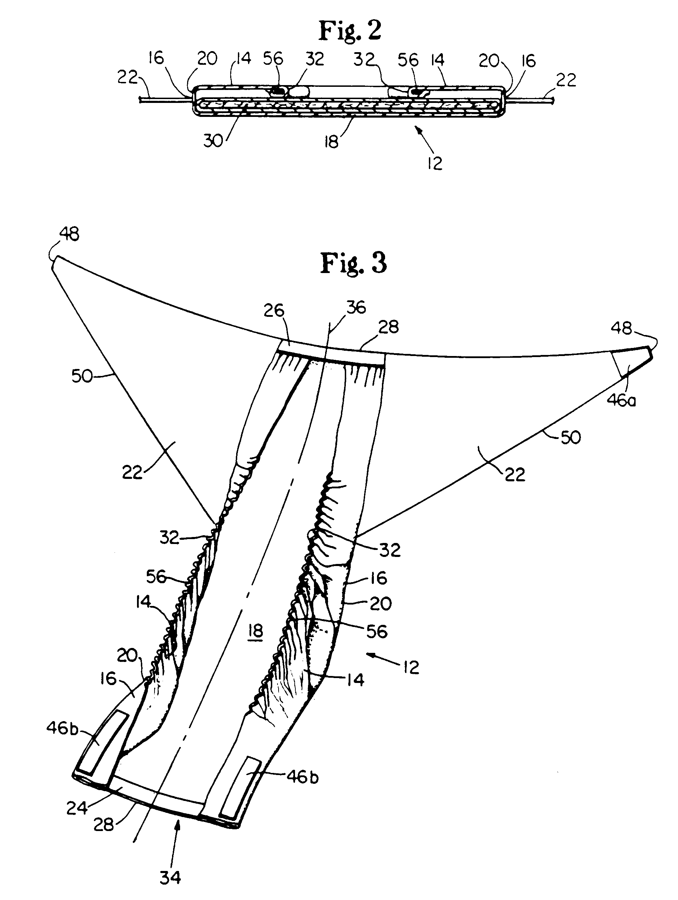

The distal edge 32 of the flaps 14 monotonically taper inwardly, towards the longitudinal centerline 36, as the rear waist margin 26 of the garment 12 is longitudinally approached. The taper provides less overlap of the flaps 14 on the absorbent core 30 near the front of the garment 12 and more overlap on the core 30 near the rear of the garment 12. This arrangement provides a larger target area for urine accumulation at the front of the garment 12 and improved capture and retention of the core 30 at the rear of the garment 12. As shown by Figure 2, the garment 12 has a substantially C-shaped cross section which provides capture and alignment of the removable core 30, making the garment 12 easier to apply and secure to the wearer.

Referring back to Figure 1 and examining each component of the garment 12 in more detail, one notes that the body portion 34 of the garment 12 is substantially symmetrically disposed about the longitudinal centerline 36 of the garment. The garment 12 as herein described is laid out in the flat, uncontracted configuration of Figure 1 unless otherwise specified.

As used herein, the term "garment" refers to an article worn about the lower torso, typically by an incontinent person, for the collection of bodily discharges. The term "longitudinal" refers to an imaginary line, axis or direction of the garment 12, which line, axis or direction extends between the transverse edges 28 of the garment 12 and is generally aligned with the vertical plane which bisects the standing wearer into left and right bodily halves. The word "lateral" refers to an imaginary line, axis or direction generally orthogonal to the longitudinal line, axis or direction and is generally sideways aligned when the garment 12 is worn.

The body portion 34 may be of any shape suitable for holding an absorbent core 30 about the anus and genitalia of the wearer. The term "body portion" refers to the assembly of a center panel 18 and one or more flaps 14 joined to the center panel 18 and typically, but not necessarily, longitudinally coextensive of the center panel 18. The body portion 34 is of a quadrilateral shape, preferably a rectangle, so that each side is substantially symmetric about the centerline 36 and a mirror image of the other side. The body portion 34 also has a greater longitudinal dimension than lateral dimension, to provide full coverage from the front waist margin 24 to the rear waist margin 26 around the lower abdominal region and torso of the wearer. The garment 12 described herein is sized to a wearer having a measurement ranging from 86.4 centimeters to 101.6 centimeters around the waist or hips, whichever measurement is larger. It is to be understood by one skilled in the art that the garment 12 described herein would have to be appropriately scaled for a larger or smaller wearer.

For the embodiment described herein, an unassembled body portion 34 having a longitudinal dimension of 76.2 centimeters, a lateral dimension at the front waist margin 24 of 38.1 centimeters and a lateral dimension at the rear waist margin 26 of 45.7 centimeters has been found suitable. The term "waist margin" refers to that portion of the garment 12 which includes and is adjacent either transverse edge 28 of the garment 12 and is generally at the highest elevation of the garment 12 while it is worn. The front waist margin 24 and rear waist margin 26 are to the front and rear of the wearer respectively as the garment 12 is worn. The difference in lateral dimensions between the front waist margin 24 and rear waist margin 26 provides for flaps 14 having nonparallel tapered distal edges 32 when the flaps 14 are folded about a line parallel to the longitudinal centerline 36.

To provide flaps 14 of a suitable geometry, each longitudinal edge 20 of the body portion 34 is folded about the line 20 parallel to the longitudinal axis 36 and disposed 12.7 centimeters from the longitudinal axis 36 towards the longitudinal edge 20 of the center panel 18. The term "flap" refers to the component of the garment 12, specifically the body portion 34, which overlays the core 30. The fold line becomes the juncture of the proximal edge 16 of the flap 14 and the longitudinal edge 20 of the body portion 34. When the flap 14 is folded about this fold line, the distal edge 32 of the flap 14 will overlay the center panel 18 by 5.1 centimeters at the front waist margin 24 and monotonically taper in a generally rectilinear fashion to an 8.9 centimeters overlap at the rear waist margin 26. The term "monotonic taper" refers to the relative change in position of a line, such as the distal edge 32 of a flap 14, having a lateral distance from the longitudinal axis 36, which lateral distance increases, decreases or remains constant as a function of the longitudinal position on the garment, and which lateral distance does not both increase and decrease as a function of the longitudinal position of the garment. Alternatively stated, the lateral distance between the centerline 36 and distal edge 32 either increases or decreases, without an inflection point, as either waist margin is longitudinally approximated. Particularly, as applied to the present invention, the flaps 14 monotonically converge as the rear waist margin 26 is approached, so that the maximum lateral distance from the distal edge 32 of a flap 14 to the centerline 36 occurs at the point of the distal edge 32 at the transverse edge 28 of the first waist margin 24.

The center panel 18 is defined by longitudinal edges 20 and transverse edges 28 and is generally symmetric about the longitudinal centerline 36. The term "center panel" refers to the component of the garment 12, specifically the body portion 34, through which the longitudinal centerline 36 passes and that supports the core 30 within the body portion 34.

The body portion 34 should be soft, flexible and stretchable to provide comfort to the wearer. The term "flexible" refers to materials which are generally compliant and readily conform to the shape and contours of the human body. Preferably, the body portion 34 material is not noisy, to provide discretion for the wearer. An elastomeric material is suitable, but a stretchable knitted material is preferred so that the wearer does not have the tactile sensation of a synthetic type material against his or her skin.

Minimal longitudinal body portion 34 elongation is desired, so that the garment 12 will be tight fitting in the crotch region and firmly position the absorbent core 30 against the body. Body portion 34 material having elastic elongation in the longitudinal direction ranging from approximately 0% to approximately 10% works well, with a value near the lower end of this range being preferred. The body portion 34 should have somewhat greater lateral elastic elongation so that the body portion 34 will more readily expand and accept the core 30 when it is inserted by the user and recover to firmly secure the core 30 in place within the body portion 34. Body portion material having elastic elongation in the lateral direction ranging from approximately 50% to approximately 120% works well. Lateral elastic elongation between approximately 100% and approximately 120% is particularly suitable. Material having a lateral fabric stretch of about 114 percent when tested in accordance with ASTM D 2594-72 utilizing a 76 x 398 millimeter sample cycled four times between 0 and 1360 grams force is suitable.

For the embodiment described herein, a body portion 34 made of 50% cotton, 50% polyester rib knit fabric, having a basis weight of approximately 240 g./sq. meter works well. The cotton provides for transmission of moisture from the wearer and a garmentlike feel against the skin, while the polyester provides durability and the capability to be laundered and reused. The term "durable" refers to a garment, or component thereof, intended for more than one usage or period of wearing, and which may be restored between subsequent periods of usage or wearing.

To better enable the garment 12 to fit tightly and discreetly to the wearer, elastic is provided at each waist margin 24 and 26. The elastic of the rear waist margin 26 provides elastic elongation of the garment about the back torso of the wearer, providing comfort and a secure fit during use. The elastic of the rear waist margin 26 is joined, preferably by sewing, at the transverse edge 28 of the body portion 34 which is nearer the more closely spaced flaps 14, i.e., the transverse edge 28 where the flaps 14 overlay more of the core 30. Each end of the elastic of the rear waist margin 26 elastic is joined to the juncture of the wing 22 and body portion 34.

The elastic of the rear waist margin 26 should be washable, generally noncontracting in the transverse direction and not pinch the skin of the wearer. For the embodiment described herein, waist elastic which can be stretched approximately 110 percent before reaching the elastic limit, is approximately 3.2 centimeters wide and approximately 16.5 centimeters long in the relaxed state is suitable. When joined to the garment 12, the elastic of the rear waist margin 26 is stretched to about 25.4 centimeters centered on the longitudinal centerline 36 of the garment 12 and has each end joined to a wing 22. Pajama Waist Elastic No. 1482, sold by the Dritz Corporation of New Bedford, Massachusetts has been found suitable.

The front waist margin 24 is also elasticized so that it may be laterally stretched to increase the width of the urine target zone. The waist elastic described above, laterally centered and slightly elongated when joined to the garment 12 (to provide a tight fit), but not elongated so far as to cause significant wrinkling of the body portion 34 is desired. Elastic at the front waist margin 24 having a relaxed length of about 10.2 centimeters and stretched about 50 percent, to about 15.2 centimeters when joined at the frontal transverse edge 28 of the garment 12 works well.

The garment 12 also has a means for securing the garment 12 to the wearer. The securing means must provide adjustment within the range of sizes of intended wearers, last for the intended life of the body portion 34 and not interfere with the collection and absorption of bodily discharges. More particularly, the means for securing the garment 12 to the wearer must accommodate wearers having limited manual dexterity, be readily accessible while the garment 12 is worn, remain attached throughout the intended wearing period and, further, not require undue assistance from others when the garment 12 is being applied to or removed from the wearer. The phrase "securing means" or the like refers to any component of the garment 12, or otherwise for associating the garment 12 with a wearer, in a position to collect bodily discharges.

One particularly suitable securing means is wings 22 disposed at or near the rear waist margin 26 of the garment 12 and that substantially symmetrically extend laterally beyond the longitudinal edges 20 of the body portion 34 in both directions. The wing 22 is joined, preferably by sewing, to the longitudinal edge 20 of the body portion 34, generally in juxtaposition with the proximal edge 16 of the flap 14 and longitudinally coextensive with the rear waist margin 26.

The wings 22 are attached to the body portion 32 at a juncture approximately 29.6 centimeters long and generally parallel to the longitudinal axis 36. The end of the rear waist margin 26 elastic is joined to the wing at this juncture.

The wings 22 should provide adjustment of the securing means in both the circumferential and head-to-foot orientations. One suitable fastening means which can be adapted to the wings 22 is made by Velcro U.S.A. of Manchester, New Hampshire, and utilizes two complementary, interengaging, releasably locking patches, one joined to each component, or article, to be secured together and commonly known as hook and loop fasteners. A wing 22 made of the Velcro brand loop material provides a large target area to which Velcro brand hook material 46 may be attached. The wings 22 have significant longitudinal and lateral dimensions, and therefore provide adjustment in either direction.

If desired, the first side of the wing 22 may be backed with a lining, of like geometry, which prevents the backing of thee Velcro brand loop material from binding or irritating the skin of the wearer. The term "first side" refers to the face of the garment 12, or its components, which are disposed towards the wearer while the garment 12 is worn. The lining may be of cotton, flannel or any flexible material which is tactily pleasing.

The rearward transverse edge of the wing 22 is generally rectilinear, orthogonal to the longitudinal axis 36, and longitudinally coextensive of the transverse edge 28 of the rear waist margin 26 of the garment 12. The opposite, or frontal, edge 50 of the wing 22 converges with the transverse edge 28 at a dull point disposed at the laterally outboard end 48 of the wing 22 so that the longitudinal dimension of the wing 22 diminishes as the outboard end 48 of the wing 22 is approached. The convergence, or diminution of longitudinal dimension, is an important feature of the wings 22 because this arrangement prevents the wings 22 from overlaying the core 30 near the lateral center of the garment 12 and thereby reduces occurrences of wetting of the wings 22 by urination.

A Velcro U.S.A. of Manchester, New Hampshire brand securing means having 268 grams per centimeter peel strength has been found suitable for fastening of the securing means. Loop material which accommodates this peel strength is sold by Velcro U.S.A. under Model No. 3001.

To complete the means for securing the garment 12 to the wearer, Velcro U.S.A. brand hook material 46, complementary to the loop material described and above having a width of 2.54 centimeters is joined to the garment 12. For the securing means described herein, Velcro U.S.A. Model No. 88 hook material 46 works well.

Two related and independent sets of fastening means 46 are utilized with the securing means of the garment 12 of the present invention. A first set of fastening means 46a is used to secure the wings 22 around the waist of the wearer, while a second set of fastening means 46b is used to secure the center panel 18 under the crotch of the wearer and to the wings 22. A patch of Velcro U.S.A. brand hook material 46, described above, joined to the first side of the outboard end 48 of either wing 22 and covering about 23 square centimeters is suitable for the first fastening means 46a.

The second fastening means 46b, that secures the body portion 34 to the wings 22, should provide adjustment in the longitudinal dimension, so that variations in wearers can be accommodated. Velcro U.S.A. brand hook material 46, described above, joined to the first side of the flaps 14 near the front waist margin 24 works well. Two patches of hook material 46, each having a longitudinal dimension of 9 centimeters, a lateral dimension of 2.5 centimeters and disposed near each longitudinal edge 20 are suitable.

As shown in Figure 3, the distal edge 32 of the flaps 14 may be reinforced, entirely or in part, with elastic 56 to form a barrier leg cuff which securely seals the flap 14 against the inner thigh of the wearer. The elastic reinforcement 56 also helps to hold the core 30 In the proper position to receive bodily discharges and prevent the garment 12 from being mispositioned and potentially soiled by the bodily discharges.

Preferably, the elastic 56 should not be disposed too close to the rear waist margin 26 of the garment 12, otherwise the wearer may sit on the elastic 56 and experience discomfort. Similarly, the elastic 56 should not be disposed too close to any of the fastening means 46, particularly those fastening means 46b disposed near the front waist margin 24 of the garment 12, otherwise bunching and wrinkling of the garment material near the second set of fastening means 46b may result and hinder manipulation of the fastening means 46b. Thus, the elastic 56 should be somewhat centered on the flap distal edge 32, as described above, and may be disposed 10.8 centimeters from the front waist margin 24 and 18.4 centimeters from the rear waist margin 26.

The leg cuff elastic 56 should provide a high degree of extensibility, without undue contractive force and should be under tension when joined to the garment 12. An elastic band which stretches about 240 percent before the elastic limit is reached is suitable. An elastic band 56 having a relaxed dimension of 19.1 centimeters and stretched to 47 centimeters when joined to the garment 12 has been found suitable. Elastic material 3.2 millimeters wide and sold by the Dritz Corporation of New Bedford, Massachusetts as Model No. 4096 has been found suitable.

The garment 12 may also comprise a means for preventing excessive longitudinal movement of the absorbent core 30 relative to the garment 12, particularly relative to the body portion 34. A particularly adequate means is end panels, generally oppositely disposed on the longitudinal axis 36 and juxtaposed with the front and rear waist margins 24 and 26. The term "end panel" refers to any partition which physically obstructs the core 30 from undue relative longitudinal movement. The end panels may be formed by the generally concave shape of the garment 12 (which is caused by contraction of the aforementioned barrier leg cuff elastic 56) and the longitudinal overlap of the front and rear waist margins 24 and 26 on the center panel 18. The end panels are therefore integral with both the center panel 18 and the flaps 14, and need not be independently joined to the garment 12.

The absorbent core 30 provides the means to collect and contain bodily discharges, particularly urine, deposited thereon. The term "core" refers to the component of the garment 12 which receives and retains the bodily discharges. The core 30 is disposable and should be conformable and nonirritating to the skin. The term "disposable" refers to a garment, or component thereof, Intended to be discarded after a single use, and not to be restored or reused. Suitable core 30 materials include layers of tissue, such as cellulose wadding and fibrated comminution pulp, or airfelt. The core 30 need not have a total absorbent capacity much greater than the total amount of bodily discharges to be absorbed and is preferably narrow and thin so as to be comfortable to the wearer. For the embodiment and wearer described herein, the total absorbent capacity should be no less than 780 grams.

The core 30 should be sized to register with the center panel 18 of the body portion 34 and to be easily inserted and removed from the garment 12. For the body portion 34 described herein, a quadrilaterally shaped core 30 having a greater longitudinal dimension than transverse dimension is suitable. Preferably, the core 30 is rectangular, approximately 65.2 centimeters in the longitudinal dimension and approximately 25.4 centimeters in the lateral dimension.

The core 30 is preferentially encased between a topsheet and a backsheet to prevent the absorbent materials of the core 30 from shredding and becoming detached while the garment 12 is worn and to ensure containment of the bodily discharges. This arrangement also assists in inserting and removing the core 30 as a unitary component.

The topsheet is oriented towards and contacts the body of the wearer. The topsheet should be flexible, clean in appearance, and somewhat opaque to hide bodily discharges collected in and absorbed by the core 30. The topsheet should exhibit good strikethrough and rewet characteristics, permitting bodily discharges to rapidly penetrate the topsheet to the core 30, but not flow back through the topsheet to the skin of the wearer. A perforated low density polyethylene topsheet having from 5% to 60% open area, typically approximately 25% open area, and from 0.01 to 0.05 millimeters in thickness is suitable.

The backsheet may be any flexible, fluid impervious film and prevents discharges absorbed by the core 30 from soiling the clothing and bedding of the wearer. The backsheet may also be impervious to malodorous gases generated by the absorbed bodily fluids, so that the malodors do not escape and become noticed by the wearer. A low density polyethylene backsheet from 0.01 to 0.05 millimeters in thickness has been found to work well.

The topsheet and backsheet are assembled about the intermediately disposed core 30 using means well known to one skilled In the art including, but not limited to, mechanical crimping, welding or adhesive bonding. The core 30 may then be inserted into the garment 12 with the topsheet facing the wearer and the backsheet facing the center panel 18.

The components of the garment 12 of the present invention may be joined and assembled using techniques, such as sewing, commonly known to one skilled in the art. The term "join" refers to the condition where a first member, or component of the garment 12, is affixed, or connected, to a second member, or component of the garment 12, either directly; or indirectly, where the first member or component is affixed, or connected, to a intermediate member or component which in turn is affixed, or connected, to the second member or component. The association between the first member, or component, and the second member, or component, is intended to remain for the life of the article.

A suitable assembly method is to cut a body portion 34 to form the center panel 18 and flaps 14 from a single piece of the material, described above, having a longitudinal dimension of 73.7 centimeters, a lateral dimension at the front waist margin 24 of 38.1 centimeters and a lateral dimension at the rear waist margin 26 of 45.7 centimeters. The leg cuff 56 elastic is joined to the distal edges 32 of the flaps 14 and the elastic of the front waist margin 24 is joined to the garment 12 at the front waist margin 24 at any time after the flaps are folded about the fold line 20. The wings 22 are joined to the body portion 34, adjacent the transverse edge 28 of the rear waist margin 26 and the juncture between the proximal edge 16 of the flap 14 and the longitudinal edge 20 of the center panel 18. The elastic of the rear waist margin 26 may then be stretched and pinned into position and joined to the body portion 34. Finally, the ends of the elastic of the rear waist margin 26 are joined to the wings 22.

The components of the garment 12 are preferably joined by sewing, and more preferably by machine sewing with a zig-zag stitch which provides some extensibility, to accommodate the stretching of the body portion 34 components. A zig-zag stitch approximately 3.2 millimeters wide and ranging from 2.5 to 5 stitches per centimeter (with the elastic uncontracted) is suitable. The type of thread utilized is not critical, however cotton covered polyester thread is commonly available and suitable.

Several variants of the present invention are feasible, the understanding of which may be enhanced if the flaps 14 of the present invention are thought of as having three trisections, a central trisection intermediate two longitudinally outer trisections. While any three points on the distal edges 32 of the flaps 14 of the embodiment of Figure 1 appear colinear, the benefits of the present invention may be recognized without flaps 14 having rectilinear distal edges 32, i.e. the flaps 14 need not taper throughout the entire length of the distal edge 32.

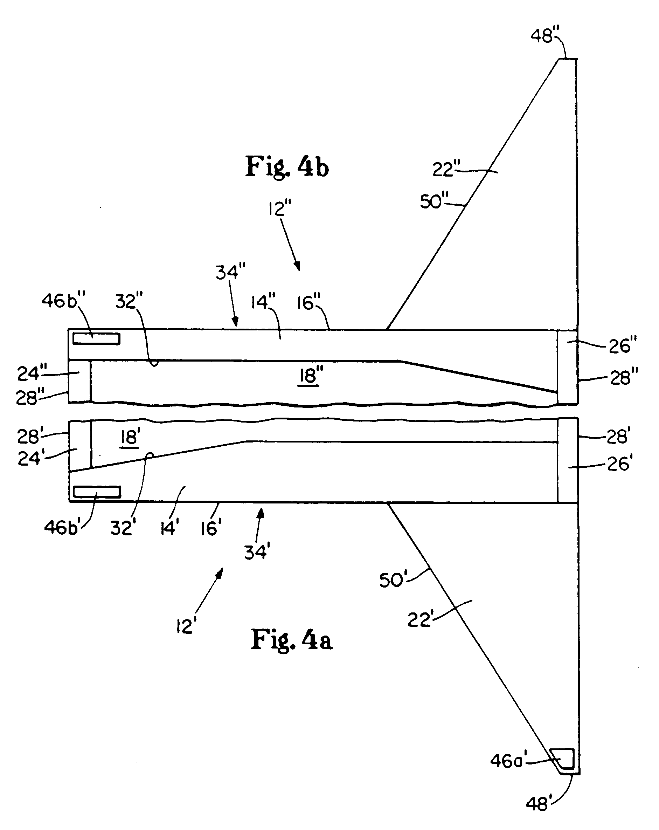

As illustrated by the left hand flap 14' of the embodiment of Figure 4a, the distal edges 32' of the flap 14' may be mutually parallel for a portion of the longitudinal dimension of the flaps 14', such portion representing the central trisection and one longitudinally outer trisection, and taper laterally outwardly (away from the longitudinal centerline 36' not shown) throughout the other longitudinally outer trisection as the front waist margin 24' of the garment 12' is longitudinally approached. This arrangement provides the advantage that a greater longitudinal portion (approximately two trisections) of the flap 14' overlays the core 30', yet the core 30' (not shown) Is still substantially uncovered in the urine acquisition zone.

Another variant, illustrated by the right hand flap of the embodiment of Figure 4b, shows the distal edges 32'' of the flaps 14'' to be mutually parallel for a length representing the central trisection and one longitudinally outer trisection, and taper laterally inwardly (towards the longitudinal centerline 36'' not shown) throughout the longitudinally outer trisection, as the rear waist margin 26'' of the garment 12'' is longitudinally approached. This arrangement provides the advantage that the wide urine target zone is provided throughout a longitudinally emphasized area (approximately two trisections) of the garment 12'' and the flaps 14'' overlay a greater portion of the core 30'' (not shown) only, as necessary, in a longitudinally deemphasized area (approximately one trisection) near the rear waist margin 26''.

A characteristic common to each of the embodiments of Figure 4 is that the flaps 14' and 14'' monotonically taper laterally away from the longitudinal centerline 36' and 36'' as the front waist margin 24' and 24'' of the garment 12' and 12'' is approached. Also, the flaps 14' and 14'' of either embodiment of Figure 4 are mutually parallel for approximately two adjacent trisections, the central trisection and one longitudinally outer trisection. The lateral distance from the point on the longitudinally outer trisection at the front waist margin 24' and 24'' of Figures 4a and 4b to the centerline is greater than the lateral distance from at least one point on one longitudinally outer trisection or the central trisection to the centerline.

While the left hand flap 14' and right hand flap 14'' of Figures 4a and 4b are shown to be mutually parallel to the symmetrically opposite flaps 14' and 14'' (not shown) for approximately two trisections and to be tapered for approximately one trisection, it is to be understood that the vertex of the distal edges 32' and 32'' of either embodiment of Figure 4 is to be longitudinally adjusted and positioned, as needed, to balance the opposing factors noted above, so that adequate core 30' and 30'' retention and a sufficient urine acquisition zone are provided. It is also understood that minor undulations In the outboard trisections of the flaps 14' and 14'' of either embodiment of Figure 4 which laterally taper are acceptable and contemplated by the present invention, so long as any point on the distal edges 32' and 32'' of such trisection has a different lateral distance to the centerline than the lateral distance from any point on the distal edge 32' and 32'' of the central trisection to the centerline 36' and 36''. Similarly, minor undulations in flaps 14 of Figure 1 are contemplated. It is to be further understood that the included angles of the nonparallel portions of the flaps 14' and 14'' of either embodiment of Figures 4a and 4b and the embodiment of Figure 1 are to be adjusted, as necessary, to balance the same opposing factors.

To apply and secure the garment 12 to a wearer, a clean and sanitary core 30 is inserted into the body portion 34 (under the flaps 14). The wearer stands, holding one wing 22 of the garment 12 in each hand with the body portion 34 and core 30 behind the wearer and downwardly oriented. The wearer encircles his or her waist with the wings 22 and secures the wings 22 together using the first set of fastening means 46a. The body portion 34 and core 30 are then brought between the legs of the wearer and secured to the second side of the wings 22, in front of the wearer with the second set of fastening means 46b. The term "second side" refers to the face of the garment 12 which is disposed towards the outer clothing of the wearer while the garment 12 is being worn.

The front waist margin 24 of the center panel 18 should be laterally stretched, to provide the maximum urine target zone width and area. If the wearer is unable to stand without difficulty, the garment 12 may be placed on a chair or bed and secured to the wearer, generally as described above, while the wearer sits or lies on the garment 12.

The distal edges 32 of the flaps 14 may be made curvilinear, rather than rectilinear. The monotonic taper of the flap distal edge 32 may be provided by either the shape of the flap 14 (as shown) or the shape of the center panel 18, i.e. by folding each flap 14 about a line which is not parallel to the longitudinal axis 36. The flops 14' and 14'' of Figures 4a and 4b may be tapered for approximately two trisections and mutually parallel for approximately one trisection. Alternative fastening means are available and the garment 12 may be made disposable rather than durable or the core 30 may be made durable rather than disposable.

## Claims

1. A garment (12) for holding a separate and removable absorbent core (30), said garment having a longitudinal centerline (36), and comprising:
a center panel (18) of generally quadrilateral shape, generally symmetrically disposed about the longitudinal centerline (36) and having a greater longitudinal than lateral dimension, said center panel (18) being formed with a front waist margin (24) and a rear waist margin (26);
two oppositely disposed flaps (14) extending longitudinally of the center panel (18) from the front waist margin (24) to the rear waist margin (26), each said flap having a proximal edge (16) and a distal edge (32), said proximal edge (16) being affixed to a longitudinal side of said center panel (18), said distal edge (32) being disposed towards the longitudinal centerline (36) whereby the lateral distance from the centerline (36) to said distal edge (32) is less than the lateral distance from the centerline (36) to said proximal edge (16), said distal edge (32) being tri-sectional, with a central section disposed intermediate two longitudinally outer sections, each said flap (14) optionally being provided along its distal edge (32) with elastic (5,6) means along at least the central section; and
integral means (22,46a,46b,48) for securing said garment (12) about the waist of a wearer,
characterised in that the distal edge (32) of each said flap (14) is formed so as to be convergent monotonically with the longitudinal center line (36) over at least one of its sections in a direction towards the rear waist margin (26), the distal edge (32) of any not convergent section being disposed parallel to the longitudinal center line (36).

2. A garment (12) according to claim 1 wherein the convergence of the distal edge (32) of each said flap (14) is provided by one of said flap (14) and said center panel (18) being generally monotonically tapered substantially throughout its length from the front waist margin (24) to the rear waist margin (26).

3. A garment (12) according to either one of claims 1 or 2 further comprising a means for preventing excessive relative longitudinal movement of an absorbent core (30) held by said garment (12).

4. A garment (12) according to claim 3 characterized in that said means for preventing excessive longitudinal movement comprises an end panel intermediate said proximal edge (16) of said flaps (14) and generally laterally coextensive with said center panel (18).

5. A garment (12) according to any one of claims 1-4 in combination with an absorbent core (30) held within and by said garment (12) solely by frictional forces.

## Patentansprüche

1. Ein Kleidungsstück (12) zum Halten eines gesonderten und entfernbaren absorbierenden Kerns (30), wobei das genannte Kleidungsstück eine Längsmittellinie (36) umfaßt und aufweist:
ein Mittelfeld (18) von allgemein trapezoider Form, welches allgemein symmetrisch um die Längsmittellinie (36) angeordnet ist und eine größere Längs- als Querdimension aufweist, wobei das genannte Mittelfeld (18) mit einem vorderen Taillenrand (24) und einem hinteren Taillenrand (26) ausgebildet ist;
zwei gegenüberliegend angeordnete Lappen (14), welche sich der Länge des Mittelfelds (18) nach vom vorderen Taillenrand (24) zum hinteren Taillenrand (26) erstrecken, wobei jeder Lappen einen proximalen Rand (16) und einen distalen Rand (32) aufweist, der genannte proximale Rand (16) an einer Längsseite des genannten Mittelfeldes (18) fixiert ist, der genannte distale Rand (32) gegen die Längsmittellinie (36) angeordnet ist, wodurch der Querabstand von der Mittellinie (36) zum genannten distalen Rand (32) geringer ist als der Querabstand von der Mittellinie (36) zum genannten proximalen Rand (16), der genannte distale Rand (32) in drei Teilstücke geteilt ist, wobei ein mittleres Teilstück zwischen zwei der Länge nach äußeren Teilstücken angeordnet ist, jeder genannte Lappen (14) gegebenenfalls entlang seinem distalen Rand (32) mit elastischen Mitteln (5,6) entlang mindestens dem zentralen Teilstück versehen ist; und
integrale Mittel (22,46a,46b,48) zum Fixieren des genannten Kleidungsstückes (12) um die Taille eines Trägers,
**dadurch gekennzeichnet,** daß der distale Rand (32) eines jeden genannten Lappens (14) so ausgebildet ist, daß er über mindestens eines seiner Teilstücke in einer Richtung gegen den hinteren Taillenrand (26) gleichförmig konvergierend mit der Längsmittellinie (36) ist, wobei der distale Rand (32) eines jeden nicht konvergierenden Teilstücks parallel zur Längsmittellinie (36) angeordnet ist.

2. Ein Kleidungsstück (12) nach Anspruch 1, bei welchem die Konvergenz des distalen Randes (32) eines jeden genannten Lappens (14) von einem von beiden, dem genannten Lappen (14) und dem genannten Mittelfeld (18), beigestellt ist, welches im wesentlichen über seine Länge vom vorderen Taillenrand (24) zum hinteren Taillenrand (26) allgemein gleichförmig spitz zulaufend ist.

3. Ein Kleidungsstück (12) nach einem der Ansprüche 1 oder 2, welches weiters ein Mittel umfaßt, um übermäßige relative Längsbewegung eines absorbierenden Kerns (30), welcher vom genannten Kleidungsstück (12) gehalten ist, zu verhindern.

4. Ein Kleidungsstück (12) nach Anspruch 3, **dadurch gekennzeichnet,** daß das genannte Mittel zum Verhindern von übermäßiger Längsbewegung ein Endfeld umfaßt, welches zwischen dem genannten proximalen Rand (16) der genannten Lappen (14) und allgemein quer koextensiv mit dem genannten Mittelfeld (18) ist.

5. Ein Kleidungsstück (12) nach einem der Ansprüche 1 bis 4, in Kombination mit einem absorbierenden Kern (30), welcher innerhalb des genannten Kleidungsstücks (12) und von diesem lediglich durch Reibungskräfte gehalten ist.

## Revendications

1. Un vêtement (12) pour maintenir une âme absorbante séparée et susceptible d'être enlevée (30), ledit vêtement ayant une ligne médiane longitudinale (36), et comprenant:
un panneau central (18) généralement en forme de quadrilatère, disposé généralement symétriquement autour de la ligne médiane longitudinale (36) et ayant une dimension longitudinale plus grande que la dimension latérale, ledit panneau central (18) étant formé d'une lisière de ceinture avant (24) et d'une lisière de ceinture arrière (26);
deux rabats disposés à l'opposé (14) s'étendant longitudinalement du panneau central (18) à partir de la lisère de ceinture avant (24) vers la lisière de ceinture arrière (26), chacun desdits rabats ayant un bord proximal (16) et un bord distal (32), ledit bord proximal (16) étant fixé au côté longitudinal dudit panneau central (18), ledit bord distal (32) étant disposé vers la ligne médiane longitudinale (36) grâce à quoi la distance latérale à partir de la ligne médiane (36) jusqu'audit bord distal (32) est inférieure à la distance latérale à partir de la ligne médiane (36) jusqu'audit bord proximal (16), ledit bord distal (32) comportant trois sections, une section centrale disposée entre deux sections longitudinalement extérieures, chacun desdits rabats (14) étant éventuellement disposé le long de son bord distal (32) avec un moyen élastique (5, 6) le long d'au moins la section centrale; et
des moyens solidaires (22, 46a, 46b, 48) pour fixer ledit vêtement (12) autour de la taille de l'utilisateur,
caractérisé en ce que le bord distal (32) de chacun desdits rabats (14) est formé de manière à converger uniformément avec la ligne médiane longitudinale (36) au moins sur une de ses sections dans une direction dirigée vers la lisière de ceinture arrière (26), le bord distal (32) de toute section non convergente étant disposé parallèlement à la ligne médiane longitudinale (36).

2. Un vêtement (12) selon la revendication 1, dans lequel la convergence du bord distal (32) de chacun desdits rabats (14) est assurée par l'un desdits rabats (14) et ledit panneau central (18) étant généralement effilé uniformément pratiquement sur toute sa longueur à partir de la lisière de ceinture avant (24) jusqu'à la lisière de ceinture arrière (26).

3. Un vêtement (12) selon l'une quelconque des revendications 1 ou 2, comportant, en outre, un moyen pour empêcher un déplacement excessif dans le sens de la longueur d'une âme absorbante (30) maintenue par ledit vêtement (12).

4. Un vêtement (12) selon la revendication 3, caractérisé en ce que ledit moyen pour empêcher le déplacement excessif dans le sens longitudinal comprend un panneau d'extrémité entre ledit bord proximal (16) desdits rabats (14) et qui s'étend généralement latéralement sur la même distance que ledit panneau central (18).

5. Un vêtement (12) selon l'une quelconque des revendications 1-4 en combinaison avec une âme absorbante (30) maintenue dans et par ledit vêtement (12) uniquement à l'aide des forces de frottement.
